(19) **Europäisches Patentamt / European Patent Office / Office européen des brevets**

(11) **EP 3 558 278 B1**

(12) **EUROPEAN PATENT SPECIFICATION**

(45) Date of publication and mention
of the grant of the patent:
**14.07.2021 Bulletin 2021/28**

(21) Application number: **17828837.9**

(22) Date of filing: **20.12.2017**

(51) Int Cl.:
*A61K 9/72* (2006.01)  *A61K 47/10* (2017.01)
*A61K 31/352* (2006.01)  *A61K 31/192* (2006.01)
*A61P 29/00* (2006.01)  *A61P 25/04* (2006.01)

(86) International application number:
**PCT/DK2017/050446**

(87) International publication number:
**WO 2018/113888 (28.06.2018 Gazette 2018/26)**

(54) **LIQUID CANNABINOID COMPOSITION**

FLÜSSIGE CANNABINOIDZUSAMMENSETZUNG

COMPOSITION DE CANNABINOÏDE LIQUIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.12.2016 DK PA201671027**

(43) Date of publication of application:
**30.10.2019 Bulletin 2019/44**

(73) Proprietor: **NordicCan A/S
7100 Vejle (DK)**

(72) Inventors:
• **SCHOU, Søren Christian
7400 Herning (DK)**

• **BRUUN, Heidi Ziegler
7120 Vejle Øst (DK)**

(74) Representative: **Kanved, Nicolai
Kanved Patent Consulting ApS
Advice House
Lysholt Allé 10
7100 Vejle (DK)**

(56) References cited:
WO-A1-2017/158539    WO-A2-2004/026857
US-A1- 2015 105 455    US-A1- 2016 000 843
US-A1- 2016 106 705

## Description

### FIELD OF INVENTION

The present invention related to the field of liquid cannabinoid compositions for use in personal vaporizers, and particularly for such liquid cannabinoid compositions comprising a large amount of a tetrahydrocannabinolic compound and/or a cannabidiolic compound present in acid form.

### BACKGROUND

In recent years, two main active compounds of cannabis, THC and CBD, have shown to have medical benefits, e.g. in respect of pain relief. On the other hand, the sale and use of cannabis is regulated in many countries and therefore the use thereof, even for medical purposes, is not always legal. While some legalized products have emerged, the scientifically based knowledge about the effects of THC and CBD is still being expanded.

One challenge is that a wide range of cannabis sources are available, and that the content and relative distribution of contents, including the active compounds THC and CBD may vary quite significantly depending on the specific cannabis source.

Use of various cannabis extracts is known for use in personal vaporizers. This has the advantage that many harmful substances inhaled during smoking of cannabis products may be avoided or at least decreased. Also, it may be a more reliable way of administrating cannabinoids, compared to smoking of cannabis products.

However, a problem still remains in obtaining pulmonary delivery form of THC and/or CBD, which can deliver a consistent of pharmacologically active THC and/or CBD and be used regulated medical purposes.

It is an object of the present invention to solve the above problems.

### SUMMARY

The invention relates to a liquid cannabinoid composition,
said liquid cannabinoid composition comprising
a vaporizer carrier liquid, and
a tetrahydrocannabinolic compound and/or a cannabidiolic compound,
wherein at least 95 percent by weight of said tetrahydrocannabinolic compound and/or cannabidiolic compound is present in acid form.

A significant advantage of the invention may be that the stability of the tetrahydrocannabinolic compound and/or cannabidiolic compound may be kept at a very high level due to a very low content of the unstable active form. This is realized by a process of separating the acid form from the active form. In more detail, the acid form of the tetrahydrocannabinolic compound and/or cannabidiolic compound may be selectively extracted by means, for example by means of reaction with a basic compound to increase the solubility of the deprotonated tetrahydrocannabinolic compound and/or cannabidiolic compound in polar solutions, such as water. By means of this process of increasing the acid form content, a very high content of acid form may be obtained, leading to a very high stability. In other words, instead of the active form, a high content of tetrahydrocannabinolic compound and/or cannabidiolic compound present in acid form was used. The acid form is convertible to the active form, e.g. by means of heat.

Also, one important advantage of the invention may be that the handling safety of the liquid cannabinoid composition may be drastically increased, both during manufacturing and for users. This is done by avoiding tetrahydrocannabinolic compound and/or cannabidiolic compound in active form, or at least keeping the contents of such at a relatively low level. When handling the liquid cannabinoid composition, e.g. in production or during administration, a person may accidentally cause a spill of liquid cannabinoid composition, e.g. by breaking a container of liquid cannabinoid composition. If the liquid cannabinoid composition comes into contact with the skin, there may be a danger that the tetrahydrocannabinolic compound and/or cannabidiolic compound is/are absorbed across the skin. However, by avoiding the use of the active form of the tetrahydrocannabinolic compound and/or cannabidiolic compound, the effect of such absorbed tetrahydrocannabinolic compound and/or cannabidiolic compound may be minimized.

One further important advantage of the invention may be that the tetrahydrocannabinolic compound and/or cannabidiolic compound may be provided as a separate component, i.e. with a low or even substantially without any residual compounds from cannabis. Thereby, the predictability of any administration of the tetrahydrocannabinolic compound and/or cannabidiolic compound and the effect thereof may be improved. Typically, different kinds of cannabis may comprise various further compounds beyond tetrahydrocannabinolic compound and/or cannabidiolic compound, hereunder non-cannabinoid substances. These compounds be present in various amounts and comprise various elements depending on the type of cannabis. In some cases, the extraction of the tetrahydrocannabinolic compound and/or cannabidiolic compound may results in a concentration of such further compounds, i.e. an amplification of any effects thereof. Further to any effects of these residual compounds, their varying presence lead to a variation in the content of the tetrahydrocannabinolic compound and/or cannabidiolic compound. Therefore, by keeping such residual compounds at a sufficiently low level or completely avoiding these, a liquid cannabinoid composition may be obtained which has a more predictable effect, i.e. for example leads to a more predicable plasma concentration of the tetrahydrocannabinolic compound and/or cannabidiolic compound.

According to the present invention said liquid cannabinoid composition comprises tetrahydrocannabinolic compound and/or cannabidiolic compound present in acid form in an amount of at least 95 percent by weight of the total amount of said tetrahydrocannabinolic compound and/or cannabidiolic compound. I.e. the amount of said tetrahydrocannabinolic compound and/or cannabidiolic compound being present in active form (i.e. THC or CBD) is relatively small, below 5 percent by weight of the total amount of THC, THCA, CBD, and CBDA. In the terms of a fraction, this means that

$$\frac{m(THCA) + m(CBDA)}{m(THC) + m(THCA) + m(CBD) + m(CBDA)} > 0.95$$

where $m(X)$ is the mass of the cannabinoid $X$.

According to the invention less than 5 percent by weight of said tetrahydrocannabinolic compound and/or cannabidiolic compound is present in active form.

It should be understood that while the embodiments of the present invention provide a liquid cannabinoid composition for pulmonary administration, some of the liquid cannabinoid composition may typically still be deposited on the oral mucosa during use, and thus some oral intake may take place. Here it should be mentioned, that controlling the size of the aerosols may assist in controlling the administration to the lungs, i.e. to obtain a high degree of pulmonary administration.

As used herein the term "THC" is intended to mean the pharmaceutically active form of tetrahydrocannabinol, i.e. (-)-trans-delta9-tetrahydrocannabinol.

As used herein the term "THCA" is intended to mean tetrahydrocannabinolic acid, i.e. the carboxylated version of THC, and encompassing both the THCA-A and THCA-B acids. Thus, THCA should be distinguished from THC, which may be obtained from THCA by a process of decarboxylation. THCA is intended to cover both the carboxylic acid form as well as the conjugated base form.

As used herein the term "CBD" is intended to mean the pharmaceutically active form of cannabidiol.

As used herein the term "CBDA" is intended to mean cannabidiolic acid, i.e. the carboxylated version of CBD. Thus, CBDA should be distinguished from CBD, which may be obtained from CBDA by a process of decarboxylation. CBDA is intended to cover both the carboxylic acid form as well as the conjugated base form.

As used herein the term "tetrahydrocannabinolic compound" is intended to cover only THCA and THC, but not other compounds, such as e.g. tetrahydrocannabivarin (THCV).

As used herein the term "cannabidiolic compound" is intended to cover only CBDA and CBD, but not other compounds.

As used herein the term "tetrahydrocannabinolic compound and/or cannabidiolic compound" is used as a common term for the tetrahydrocannabinolic compound and the cannabidiolic compound. This term allows either one of the two compounds or both compounds to be present as signified by the term "and/or".

As used herein the term "acid form" is intended to mean the carboxylated form of a cannabinoid and is to be distinguished from the active form. For example, the acid form of THC is THCA whereas the acid form of CBD is CBDA. Thus, when referring to the acid form, both the carboxylic acid form as well as the conjugated base form is intended to be covered.

As used herein the term "active form" is intended to mean the decarboxylated form of a cannabinoid and is to be distinguished from the acid form. Examples of cannabinoids in active form includes *THC* and *CBD.*

As used herein the term "vaporizer carrier liquid" is intended to refer to a carrier liquid suitable for use in a personal vaporizer. One important limitation in embodiments of the invention is that the vaporizer carrier liquid must be pharmaceutically acceptable, particularly acceptable for pulmonary administration, and may thus typically be composed from one or more pharmaceutically acceptable excipients.

As used herein the term "vaporizer" is intended to refer to a device comprising a number of parts, the vaporizer being arranged for reducing a liquid to a fine spray of droplets, i.e. a device which transforms a liquid into aerosols by means of heat. One example of a vaporizer may be a device that uses heating, such as resistive heating, to evaporate a liquid that may form aerosol upon condensation. Vaporizers may also be referred to as personal vaporizers, atomizers, or e-cigarettes, or may be a heat utilizing inhalator.

As used herein the term "propylene glycol" is intended to refer to alpha-propylene glycol, i.e. propane-1,2-diol.

According to an advantageous embodiment of the invention at least 98 percent by weight of said tetrahydrocannabinolic compound and/or cannabidiolic compound is present in acid form.

According to an advantageous embodiment of the invention at least 99 percent by weight of said tetrahydrocannabinolic compound and/or cannabidiolic compound is present in acid form.

According to an advantageous embodiment of the invention less than 4 percent by weight of said tetrahydrocannabinolic compound and/or cannabidiolic compound is present in active form.

According to an advantageous embodiment of the invention less than 2 percent by weight of said tetrahydrocannabinolic compound and/or cannabidiolic compound is present in active form.

According to an advantageous embodiment of the invention less than 1 percent by weight of said tetrahydrocannabinolic compound and/or cannabidiolic compound is present in active form.

According to an advantageous embodiment of the invention said tetrahydrocannabinolic compound and/or a cannabidiolic compound is present in an amount of between 0.01 and 25 percent by weight of said liquid cannabinoid composition, such as between 1 and 10 percent by weight of the liquid cannabinoid composition.

According to an embodiment of the invention said tetrahydrocannabinolic compound and/or said cannabidiolic compound is present in an amount of between 0.1 and 25 percent by weight of said liquid cannabinoid composition.

According to an embodiment of the invention said tetrahydrocannabinolic compound and/or said cannabidiolic compound is present in an amount of between 1 and 15 percent by weight of said liquid cannabinoid composition.

According to an embodiment of the invention said tetrahydrocannabinolic compound and/or said cannabidiolic compound is present in an amount of between 2 and 8 percent by weight of said liquid cannabinoid composition.

According to an embodiment of the invention said tetrahydrocannabinolic compound and/or said cannabidiolic compound is present in an amount of between 1 and 5 percent by weight of said liquid cannabinoid composition.

According to an advantageous embodiment of the invention said liquid cannabinoid composition comprises non-cannabinoid terpenoids in an amount of less than 5 percent by weight.

In other words, the liquid cannabinoid composition of the above embodiment comprises from 0 to 5 percent by weight of terpenoids, Thus, the liquid cannabinoid composition may be free of terpenoids or may comprise terpenoids in amounts up to 5 percent by weight, e.g. in amounts of 0.01 to 5 percent.

One advantage of the above embodiment may be that a liquid cannabinoid composition with a higher predictability may be obtained, particularly a higher predictability with respect to the pharmaceutical effect after administration.

Examples of non-cannabinoid terpenoids may include beta-myrcene, beta-caryophyllene, limonene, linalool, pulegone, 1,8-cineole, alpha-pinene, alpha-terpineol, terpinen-4-ol, 4-terpineol, p-cymene, borneol, delta3-carene.

Examples of phytosterols may include beta-sitosterol.

According to an advantageous embodiment of the invention said liquid cannabinoid composition comprises non-cannabinoid terpenoids in an amount of between 0 and 4 percent by weight.

According to an advantageous embodiment of the invention said liquid cannabinoid composition comprises non-cannabinoid terpenoids in an amount of between 0 and 2 percent by weight.

According to an advantageous embodiment of the invention said liquid cannabinoid composition comprises non-cannabinoid terpenoids in an amount of between 0 and 1 percent by weight.

According to an advantageous embodiment of the invention said liquid cannabinoid composition is substantially free of non-cannabinoid terpenoids.

According to an advantageous embodiment of the invention said tetrahydrocannabinolic compound and/or cannabidiolic compound is/are added at as a cannabinoid acid composition comprising at least 95 percent by weight of said tetrahydrocannabinolic compound and/or cannabidiolic compound.

The content of said tetrahydrocannabinolic compound and/or cannabidiolic compound may be verified by means of high-performance liquid chromatography (HPLC).

According to an advantageous embodiment of the invention said liquid cannabinoid composition comprises non-cannabinoid terpenoids, flavonoids, and phytosterols in a total amount of less than 5 percent by weight.

In other words, the liquid cannabinoid composition of the above embodiment comprises from 0 to 5 percent by weight of terpenoids, flavonoids, and phytosterols. Thus, the liquid cannabinoid composition may be free of one, more, or all of terpenoids, flavonoids, and phytosterols, or the liquid cannabinoid composition may comprise terpenoids, flavonoids, and phytosterols in total amounts up to 5 percent by weight, i.e. the total amount of terpenoids, flavonoids, and phytosterols may be between 0.01 and 5 percent by weight.

According to an advantageous embodiment of the invention said liquid cannabinoid composition is substantially free of non-cannabinoid terpenoids, flavonoids, and phytosterols.

According to an advantageous embodiment of the invention said liquid cannabinoid composition comprises said tetrahydrocannabinolic compound and/or cannabidiolic compound, said vaporizer carrier liquid, and optionally flavor in a total amount of at least 90 percent by weight, such as at least 95 percent by weight, such as at least 98 percent by weight, such as at least 99 percent by weight.

According to an advantageous embodiment of the invention said tetrahydrocannabinolic compound and/or cannabidiolic compound comprise(s) at least 90 percent by weight of a tetrahydrocannabinolic compound or a cannabidiolic compound, such as at least 95 percent by weight, such as at least 98 percent by weight, such as at least 99 percent by weight.

Thus, according to the above embodiment, at least 90 percent of the tetrahydrocannabinolic compound and/or cannabidiolic compound is either a tetrahydrocannabinolic compound or a cannabidiolic compound. Thus, in the above embodiment, a 50-50 mixture of a tetrahydrocannabinolic compound and a cannabidiolic compound is excluded. The content of said tetrahydrocannabinolic or cannabidiolic compound may be verified by means of high-performance liquid chromatography (HPLC).

One advantage of the above embodiment may be that by utilizing a tetrahydrocannabinolic or cannabidiolic compound a predictable single cannabinoid composition may be obtained. The effects of single cannabinoid compositions may be simpler to determine, since no interplay between e.g. THC and CBD may occur, and thus such compositions may be simpler to document, and compliance with e.g. various national law requirements may in some cases be simplified.

According to an advantageous embodiment of the invention said tetrahydrocannabinolic compound and/or cannabidiolic compound consist(s) essentially of a tetrahydrocannabinolic compound or a cannabidiolic compound.

Thus, according to the above embodiment the liquid cannabinoid composition comprises either essentially only a tetrahydrocannabinolic compound or essentially only a cannabidiolic compound.

According to an advantageous embodiment of the invention said tetrahydrocannabinolic compound and/or cannabidiolic compound comprise(s) at least 90 percent by weight of a tetrahydrocannabinolic compound, such as at least 95 percent by weight, such as at least 98 percent by weight, such as at least 99 percent by weight.

The content of said tetrahydrocannabinolic compound may be verified by means of high-performance liquid chromatography (HPLC).

According to an advantageous embodiment of the invention said tetrahydrocannabinolic compound and/or cannabidiolic compound consist(s) essentially of a tetrahydrocannabinolic compound.

According to an advantageous embodiment of the invention said tetrahydrocannabinolic compound and/or cannabidiolic compound comprise(s) at least 90 percent by weight of a cannabidiolic compound, such as at least 95 percent by weight, such as at least 98 percent by weight, such as at least 99 percent by weight.

The content of said cannabidiolic compound may be verified by means of high-performance liquid chromatography (HPLC).

According to an advantageous embodiment of the invention said tetrahydrocannabinolic compound and/or cannabidiolic compound consist(s) essentially of a cannabidiolic compound.

In certain embodiments it may be considered advantageous to use vaporizer carrier liquids, which, when atomized are visible to the human eye, whereby they imitate the appearance of smoke from conventional cannabis products, such as joints etc.

According to an advantageous embodiment of the invention said vaporizer carrier liquid is chosen from group consisting of water; alcohols, such as ethanol, propylene glycol, polyethylene glycol such as PEG 400, glycerol, and other similar alcohols; and mixtures or combinations thereof.

According to an embodiment of the invention, the vaporizer carrier liquid comprises, if comprising water, at least one component selected from the group consisting of alcohols, such as ethanol, propylene glycol, polyethylene glycol such as PEG 400, glycerol, and other similar alcohols; and mixtures or combinations thereof.

According to an advantageous embodiment of the invention said vaporizer carrier liquid comprises a substance selected from the group alcohols, such as ethanol, propylene glycol, polyethylene glycol such as PEG 400, glycerol, and other similar alcohols; and mixtures or combinations thereof.

According to an embodiment of the invention said vaporizer carrier liquid is selected from the group of water, ethanol, propylene glycol, glycerol, PEG-400, and combinations thereof.

According to an embodiment of the invention said vaporizer carrier liquid comprises a substance selected from the group of ethanol, propylene glycol, glycerol, PEG-400, and combinations thereof.

According to an embodiment of the invention said vaporizer carrier liquid comprises a substance selected from the group of propylene glycol, glycerol, PEG-400, and combinations thereof.

According to an embodiment of the invention said vaporizer carrier liquid comprises water. Water may e.g. be present in an amount of 0.1 to 99.9 percent by weight of the liquid cannabinoid composition.

According to an embodiment of the invention said vaporizer carrier liquid comprises ethanol. Ethanol may e.g. be present in an amount of 0.1 to 99.9 percent by weight of the liquid cannabinoid composition.

According to an embodiment of the invention said vaporizer carrier liquid comprises propylene glycol. Propylene glycol may e.g. be present in an amount of 0.1 to 99.9 percent by weight of the liquid cannabinoid composition.

According to an embodiment of the invention said vaporizer carrier liquid comprises glycerol. Glycerol may e.g. be present in an amount of 0.1 to 99.9 percent by weight of the liquid cannabinoid composition.

According to an embodiment of the invention said vaporizer carrier liquid comprises polyethylene glycol, such as PEG-400. Polyethylene glycol, such as PEG-400, may e.g. be present in an amount of 0.1 to 99.9 percent by weight of the liquid cannabinoid composition.

According to an advantageous embodiment of the invention said liquid cannabinoid composition comprises said vaporizer carrier liquid in amount of 75 to 99.9 percent by weight of the liquid cannabinoid composition.

According to an embodiment of the invention said liquid cannabinoid composition comprises vaporizer said carrier liquid in amount of 80 to 98 percent by weight of the liquid cannabinoid composition, such as 85 to 95 percent by weight of the liquid cannabinoid composition.

According to an advantageous embodiment of the invention the liquid cannabinoid composition has a boiling point of between 80 and 400 degrees Celsius, such as between 100 and 250 degrees Celsius.

According to an advantageous embodiment of the invention the vaporizer carrier liquid has a boiling point of between 80 and 400 degrees Celsius, such as between 100 and 250 degrees Celsius.

According to an advantageous embodiment of the invention the liquid cannabinoid composition is for pulmonary and/or oromucosal administration.

According to an embodiment of the invention the desired administration may be influenced by controlling the size distribution of produced aerosols, e.g. the average diameter of the aerosols.

According to an advantageous embodiment of the invention the liquid cannabinoid composition is for pulmonary administration.

According to an advantageous embodiment of the invention said liquid cannabinoid composition comprises flavoring.

According to an advantageous embodiment of the invention said liquid cannabinoid composition comprises 0.01 - 10 percent by weight of flavoring, such as 0.01 - 5 percent by weight of flavoring, 0.01 - 0.5 percent by weight of flavoring.

Typically, it may be desired that the user experiences one or more flavoring sensations when using the liquid cannabinoid composition with a personal vaporizer. This may e.g. be done to mask the taste of cannabinoid(s). The flavorings may be designed to imitate a smoking experience of a cannabis product, a conventional cigarette, or may be based on other flavorings, or may combine any of these.

According to an advantageous embodiment of the invention said flavoring comprises flavoring selected from the group of almond, almond amaretto, apple, Bavarian cream, black cherry, black sesame seed, blueberry, brown sugar, bubblegum, butterscotch, cappuccino, caramel, caramel cappuccino, cheesecake (graham crust), cinnamon redhots, cotton candy, circus cotton candy, clove, coconut, coffee, clear coffee, double chocolate, energy cow, graham cracker, grape juice, green apple, Hawaiian punch, honey, Jamaican rum, Kentucky bourbon, kiwi, koolada, lemon, lemon lime, tobacco, maple syrup, maraschino cherry, marshmallow, menthol, milk chocolate, mocha, Mountain Dew, peanut butter, pecan, peppermint, raspberry, banana, ripe banana, root beer, RY 4, spearmint, strawberry, sweet cream, sweet tarts, sweetener, toasted almond, tobacco, tobacco blend, vanilla bean ice cream, vanilla cupcake, vanilla swirl, vanillin, waffle, Belgian waffle, watermelon, whipped cream, white chocolate, wintergreen, amaretto, banana cream, black walnut, blackberry, butter, butter rum, cherry, chocolate hazelnut, cinnamon roll, cola, creme de menthe, eggnog, English toffee, guava, lemonade, licorice, maple, mint chocolate chip, orange cream, peach, pina colada, pineapple, plum, pomegranate, pralines and cream, red licorice, salt water taffy, strawberry banana, strawberry kiwi, tropical punch, tutti frutti, vanilla, or any combination thereof.

According to an embodiment of the invention, the flavoring may comprise cannabis flavoring.

According to an advantageous embodiment of the invention the liquid cannabinoid composition further comprises sweetener, such as an artificial sweetener, such as sucralose.

According to an embodiment of the invention, said sweetener is included in amounts of between 0.1 and 5 percent by weight of the liquid cannabinoid composition, such as between 0.2 and 3 percent by weight of the liquid cannabinoid composition, such as between 0.5 and 2 percent by weight of the liquid cannabinoid composition.

Other sweeteners may be used, if they are sufficiently resistant to heat, and do not caramelize or otherwise degrade upon heating in the personal vaporizer.

Described is a method of obtaining a liquid cannabinoid composition,

the method comprising the steps of

providing a cannabinoid acid composition comprising a tetrahydrocannabinolic compound and/or a cannabidiolic compound,

providing a vaporizer carrier liquid,

mixing said cannabinoid acid composition with said vaporizer carrier liquid, wherein at least 95 percent by weight of the total amount of said tetrahydrocannabinolic compound and/or cannabidiolic compound is present in acid form.

Said cannabinoid acid composition comprises a tetrahydrocannabinolic compound and/or a cannabidiolic compound in an amount of at least 90 percent by weight, such as at least 95 percent by weight, such as at least 98 percent by weight, such as at least 99 percent by weight.

Said step of providing said cannabinoid acid composition comprises the steps of

- providing a mixed acid-active composition comprising a tetrahydrocannabinolic compound and/or a cannabidiolic compound being present in a mixture of acid form and active form, and

- obtaining the cannabinoid acid composition by separation of the acid form from the active form.

Said step of providing said cannabinoid acid composition comprises extracting from cannabis said mixed acid-active composition.

Said step of extracting said mixed acid-active composition comprises extracting a tetrahydrocannabinolic compound and/or a cannabidiolic compound from cannabis.

Further described is a method of activating a liquid cannabinoid composition, the method comprising the steps of providing the liquid cannabinoid composition according to the invention or any of its embodiments,

aerosolizing the liquid cannabinoid composition by means of a personal vaporizer to obtain aerosols comprising said tetrahydrocannabinolic compound and/or cannabidiolic compound,

wherein at least 50 percent by weight of said tetrahydrocannabinolic compound and/or cannabidiolic compound is present in active form in said aerosols.

The method further comprises the step of administrating said aerosols by means of inhalation.

The invention further relates to a liquid cannabinoid composition according to the invention or any of its embodiments for use as a medicament.

The invention further relates to a liquid cannabinoid composition according to the invention or any of its embodiments for use in alleviation of pain, such as neurotic pain or cancer-related pain.

The invention further relates to a liquid cannabinoid composition according to the invention or any of its embodiments for therapeutic use in mitigation of appetite deficiency.

The invention further relates to a cannabinoid container comprising the liquid cannabinoid composition according to the invention or any of its embodiments.

According to an advantageous embodiment of the invention said cannabinoid container is impermeable to oxygen.

Thus, where the cannabinoid container comprises a breakable sealing, the breakable sealing is also oxygen impermeable.

According to an advantageous embodiment of the invention said cannabinoid container comprises a breakable sealing.

For example, the breakable sealing may comprise two or more layers. In one example embodiment, the breakable sealing may comprise an inner plastic layer, e.g. of the same material as the rest of the container, and an outer foil.

According to an embodiment of the invention, the cannabinoid container is formed at least partly of plastic substance, preferably substantially free of substances, metals (Fe, Sn) e.g. that cause decarboxylation of said tetrahydrocannabinolic compound and/or cannabidiolic compound.

According to an advantageous embodiment of the invention, the cannabinoid container is formed of molded plastic.

Molding in the present context includes e.g. blowing molding or extrusion molding. A preferred molding should imply that a relatively small sized cannabinoid container may be achieved, while still providing a gas tight compartment.

According to an advantageous embodiment of the invention, the cannabinoid container is formed of inert plastic.

I.e. the cannabinoid container is formed by a plastic being inert towards its content, and does not contribute substantially to the chemical composition of its content, i.e. the liquid cannabinoid composition.

Metals are usually less gas permeable than plastics. This may be at least partly compensated for by using cannabinoid containers of plastic having a greater wall thickness than the usual metal container.

Suitable materials for the manufacture of cannabinoid containers may be acrylonitrile butadiene styrene (ABS), polystyrene (PS), polyethylene terephthalate (PET), polypropylene (PP), polyethylene (PE), polytetrafluoroethylene (PTFE), polyoxymethylene (POM), polycarbonate (PC), polymethyl methacrylate (PMMA), polyimide (PI), styrene-acrylonitrile resin (SAN), polyhydroxyethylmethacrylate (PHEMA), polydimethylsiloxane (PDMS), polyether ether ketone (PEEK), or polyamide (PA).

According to an advantageous embodiment of the invention, the cannabinoid container is formed wholly or partly of polytetrafluoroethylene (PTFE).

According to an advantageous embodiment of the invention, the cannabinoid container is formed of high density polyethylene (HDPE).

According to an advantageous embodiment of the invention, at least a part of the cannabinoid container comprises thermoplastic.

The invention further relates to a personal vaporizer comprising a cannabinoid container according to the invention or any of its embodiments.

According to an advantageous embodiment of the personal vaporizer further comprises a heating element for heating the liquid cannabinoid composition to obtain aerosols,

wherein at least 50 percent by weight of said tetrahydrocannabinolic compound and/or cannabidiolic compound is present in active form in said aerosols.

## FIGURES

The invention will now be described with reference to the figures where

Figure 1 illustrates a personal vaporizer according to an embodiment of the invention,

Figure 2 illustrates a cannabinoid container according to an embodiment of the invention, and

Figure 3 illustrates a method according to an embodiment of the invention.

## DETAILED DESCRIPTION

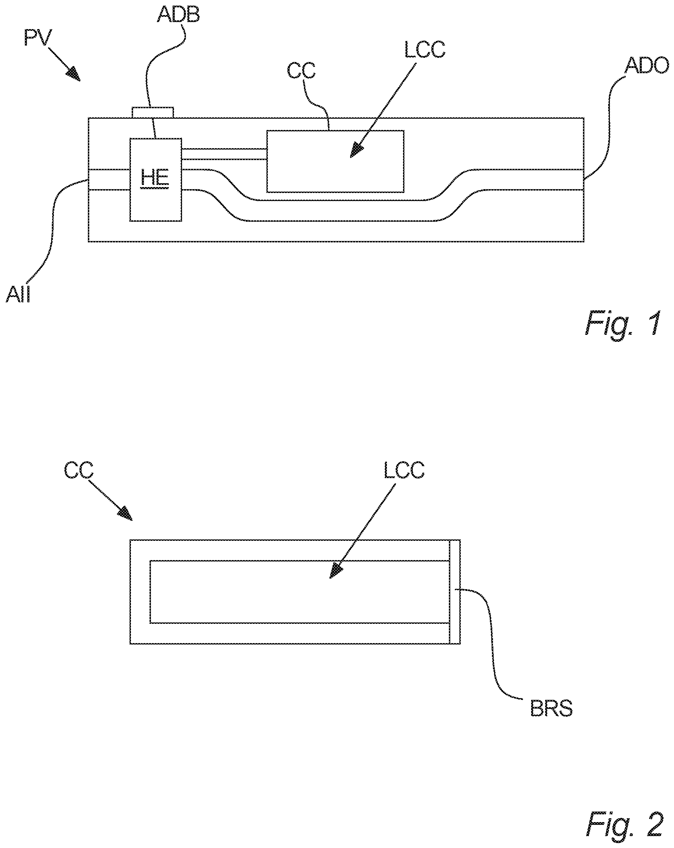

Referring to figure 1, a personal vaporizer PV according to an embodiment of the invention is illustrated.

The personal vaporizer PV comprises a cannabinoid container CC and a heating element HE.

The cannabinoid container CC contains a liquid cannabinoid composition LCC. The liquid cannabinoid composition LCC a vaporizer carrier liquid, and a tetrahydrocannabinolic compound and/or a cannabidiolic compound. At least 95 percent by weight of said tetrahydrocannabinolic compound and/or cannabidiolic compound is present in acid form.

Also, the personal vaporizer PV has an air inlet AII for taking in air and an administration outlet for letting out the air and aerosols of the liquid cannabinoid composition to facilitate pulmonary administration of the liquid cannabinoid composition.

The air inlet AII is connected to the heating element HE, which again is connected to the administration outlet ADO. Thereby, air can pass from the outside, into the air inlet AII, through the inner space of the personal vaporizer PV, via the heating element HE, and to the administration outlet ADO.

The heating element HE is arranged to receive the liquid cannabinoid composition LCC from the cannabinoid container CC, and is configured to apply heat to the liquid cannabinoid composition LCC to aerosolized this, i.e. create aerosols of the liquid cannabinoid composition. The heating element HE may be devised in various ways, for example as a coil heating a wick connected to cannabinoid container. Typically, the heating element HE is connected to a portable energy storage, such as a battery for drawing power to supply the heat. It should be mentioned that other types of heating elements may also be used in personal vaporizers PV as long as they can aerosolize the liquid cannabinoid composition so as to produce aerosols of sizes suitable for pulmonary administration.

Suitable electronic circuits (not illustrated) may be electrically connected to the administration button ADB so as to receive an electronic signal when the administration button is activated. Such electronic circuits may then activate the heating element HE, and in some embodiments also any mechanisms for drawing liquid cannabinoid composition LCC from the cannabinoid container C.

Moreover, it should be mentioned that the personal vaporizer illustrated on figure 1 is only of illustrative purposes and that the overall design may be modified e.g. to satisfy aesthetic and/or practical considerations, such as easy contact with the mouth etc.

Now, referring to figure 2, a cannabinoid container CC is illustrated according to an embodiment of the invention. As shown, the cannabinoid container CC contains the liquid cannabinoid composition LCC described in connection with the embodiment of figure 1. The cannabinoid container CC of figure 2 further comprises a breakable sealing BRS, which may be devices as a thin layer of sealing material. The sealing material may comprise one or more layers. Alternatively, the breakable sealing BRS may comprise a lid or a cap, where the removal of the lid or cap may break the sealing. The breakable sealing BRS may also comprise a combination of a thin layer of sealing material and a lid or a cap. The cannabinoid container CC may keep the liquid cannabinoid composition LCC in a protected environment until broken.

The cannabinoid container CC may then be inserted into a personal vaporizer PV, for example in such a way that the breakable sealing BRS is then broken when inserting the cannabinoid container CC. Alternatively, the breakable sealing BRS may be broken immediately before insertion of the cannabinoid container CC to avoid exposure to ambient conditions, such as e.g. oxygen.

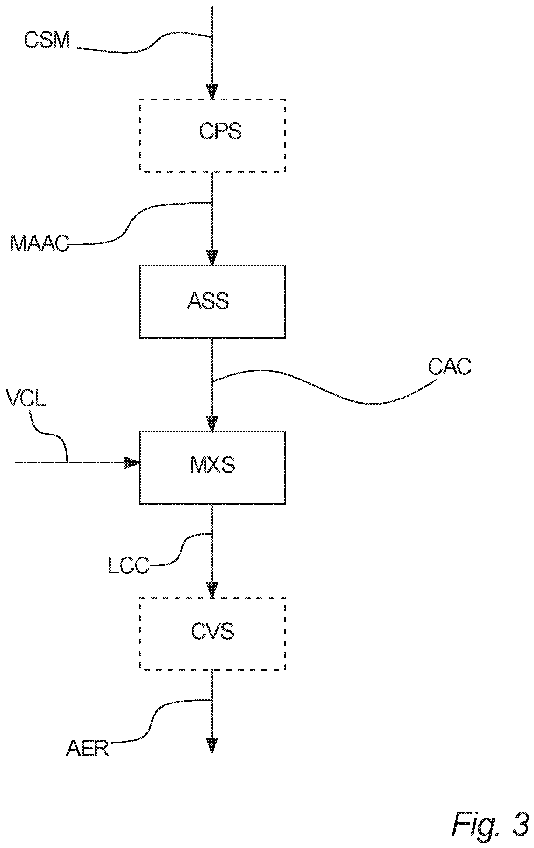

Referring to figure 3, a method is illustrated.

First, a cannabinoid starting material CSM is provided. This may be provided as cannabis, or alternatively as a cannabis extract.

Then, a mixed acid-active composition MAAC is obtained by an optional cannabinoid purifying step CPS. This step removes non-cannabinoid materials, such as terpenoids, flavonoids, phytosterols, and others. It should be understood that this removal may or may not be complete according to the specific embodiment. It may also remove cannabinoids other than tetrahydrocannabinolic and cannabidiolic compounds. In some embodiments, where only a single cannabinoid is desired, this step may remove other cannabinoids, i.e. also tetrahydrocannabinolic compounds or cannabidiolic compounds.

The cannabinoid purification step CPS may be realized by a single purification step, or may comprises a two or more sub-steps. The sub-steps may for example each address one or more specific compounds to be removed, or may also be repetitions of essentially the same step to obtain a higher concentration of the desired compound(s).

In some embodiments, the mixed acid-active composition MAAC comprises non-cannabinoid terpenoids in an amount of less than 5 percent by weight, and may thus be essentially free of terpenoids.

In some embodiments, the mixed acid-active composition MAAC comprises further substances, apart from said tetrahydrocannabinolic compound and/or cannabidiolic compound, in an amount of less than 5 percent by weight, and may thus be essentially free of further substances, apart from said tetrahydrocannabinolic compound and/or cannabidiolic compound.

The mixed acid-active composition MAAC is then subjected to an acid form separation step ASS to obtain a cannabinoid acid composition CAC. The cannabinoid acid composition CAC comprises a tetrahydrocannabinolic compound and/or a cannabidiolic compound, where at least 95 percent by weight of the total amount of said tetrahydrocannabinolic compound and/or cannabidiolic compound in the cannabinoid acid composition CAC is present in acid form.

The acid form separation step ASS may be realized by a single acid removal step, or may comprise two or more sub-steps, such as repetitive sub-steps to obtain the cannabinoid acid composition CAC.

In some embodiments the cannabinoid purification step CPS and the acid form separation step ASS is realized in a single step or single series of step, whereas in other embodiments the two steps are reversed in order.

Then, in a mixing step MXS, the cannabinoid acid composition CAC is mixed with the vaporizer carrier liquid VCL. The vaporizer carrier liquid VCL may comprise one or more of various different components, such as e.g. water; alcohols, hereunder e.g. ethanol, propylene glycol, polyethylene glycol, such as PEG 400, glycerol, and other similar alcohols; and mixtures or combinations thereof. The vaporizer carrier liquid VCL may be added as a single premixed portion, or may be added in a stepwise manner, e.g. if it comprises two or more components.

The obtained liquid cannabinoid composition LCC may then be subjected to a conversion step CVS to obtain aerosols AER comprising the active form of the comprised tetrahydrocannabinolic compound and/or cannabidiolic compound.

The conversion step CVS is performed by applying heat, for example by means of a personal vaporizer PV as described in the embodiment illustrated on figure 1.

The produced aerosols comprise at least 50 percent by weight of said tetrahydrocannabinolic compound and/or cannabidiolic compound in active form.

## LIST OF FIGURE REFERENCES

PV. Personal vaporizer
ADB. Administration button
ADO. Administration outlet
AII. Air intake
HE. Heating element
CC. Cannabinoid container
BRS. Breakable sealing
LCC. Liquid cannabinoid composition
CAC. Cannabinoid acid composition
MAAC. Mixed acid-active composition
CSM. Cannabinoid starting material
CPS. Cannabinoid purifying step
ASS. Acid form separation step
MXS. Mixing step
CVS. Conversion step
AER. Aerosols

## EXAMPLES

The following examples are illustrative of the present invention and should not be considered as limiting the scope of the invention.

### Example 1

**Cannabinoid raw materials**

Cannabinoids are available in different forms, e.g. paste, oil and crystals and in different purities. Depending on the form of cannabinoids the manufacturing steps will vary.

Tetrahydrocannabinolic compound and/or a cannabidiolic compound are extracted/prepared in a way where 95 percent by weight is present in acid form.

The tetrahydrocannabinolic compound and/or a cannabidiolic compound as acid form could also be prepared as synthetic or semi synthetic.

For the following experiment, THC and THCA as a form as thick oil, has been used.

Table 1. Different purity of THC and THCA. THCA and THC could be replaced with CBDA and/or CBD or in combination. Other = non-cannabinoid terpenoids, flavonoids, phytosterols, other cannabinoids etc.

| Sample no. | THC | THCA | Other | Total |
|---|---|---|---|---|
| 101 | 99% | | 1% | 100 |
| 102 | - | 90% | 10% | 100 |
| 103 | - | 95% | 5% | 100 |
| 104 | - | 99% | 1% | 100 |
| 105 | - | 49% | 51% | 100 |
| 106 | - | 25% | 75% | 100 |

### Example 2

**Preparation of E-liquid containing cannabinoid**

The selected cannabinoid is sample 104, THCA (thick oil).

Preparation of 100g E-liquid with 5% THCA.

5 grams THCA is weighed and added into a beaker.

15 grams of propylene glycol (PG with a purity of min. 98%, USP/Ph.Eur) is weighed and added to the beaker with THCA. The THCA is dissolved in PG using magnetic stirring until visual homogenies mixture.

The rest of PG is weighed (53 grams) and added to the beaker and stirred until visual homogenies mixture.

17 grams of vegetables glycerin (VG with a purity of min. 98%, USP/Ph.Eur) is weighed and added to the beaker and stirred until visual homogenies mixture.

10 grams of peppermint flavor (food quality suitable for vaping) is weighed and added to the beaker and stirred until visual homogenies mixture.

The E-liquid mixture containing 5% THCA is now ready to be added into e.g. 1ml cartomizers and ready to be vaped.

### Example 3

**Preparation of E-liquid containing cannabinoid**

The selected cannabinoid is sample 104, THCA (thick oil).

Preparation of 100g E-liquid with 5% THCA.

A pre-mixture is made by THCA and ethanol.

5 grams THCA is weighed and added into a beaker.

The THCA is dissolved in 10 grams of ethanol (96% purity) using magnetic stirring until visual homogenies mixture.

15 grams of propylene glycol (PG with a purity of min. 98%, USP/Ph.Eur) is weighed and added to the beaker with THCA. The THCA is dissolved in PG using magnetic stirring until visual homogenies mixture.

The rest of PG is weighed (53 grams) and added to the beaker and stirred until visual homogenies mixture.

17 grams of vegetables glycerin (VG with a purity of min. 98%, USP/Ph.Eur) is weighed and added to the beaker and stirred until visual homogenies mixture.

10 grams of peppermint flavor (food quality suitable for vaping) is weighed and added to the beaker and stirred until visual homogenies mixture.

The E-liquid mixture containing 5% THCA is now ready to be added into e.g. 1ml cartomizers and ready to be vaped.

## Example 4

**Preparation of simple E-liquid containing different types of cannabinoid**

Table 2. Composition of E-liquid with different content of THC and THCA.

| Raw Material | 107 | 108 | 109 |
|---|---|---|---|
| THC (sample 101) | 5% | 2.5% | - |
| THCA (sample 104) | - | 2.5% | 5% |
| PG | 95% | 95% | 95% |
| Total | 100 | 100 | 100 |
| The active ingredient THC = 99% THC; The active ingredient THCA = 99% THCA; The raw material PG = Propylene glycol. THCA and THC could be replaced with CBDA and/or CBD or in combination. | | | |

Preparation:

THC and THCA are dissolved in PG using magnetic stirring until visual homogenies mixture (see example 2 for further details).

The E-liquid mixture containing 5% cannabinoids is now ready to be added into 10ml plastic bottles. These 10ml plastic bottles are stored at accelerated stability conditions (40°C/75% RH) for 1 month.

The content of THC and THCA has been analyzed by HPLC after storage at 40°C/75% RH 1 month. The stability results are listed in table 3. Stability score goes from + up to +++++, the sum of + means increased stability (1+ = low stability and 5+ = high stability).

Table 3. Stability results of E-liquid after 1 month storage at 40°C/75% RH.

| Stability results of E-liquid - 1 month at 40°C/75% RH | | | |
|---|---|---|---|
| | 107 | 108 | 109 |
| Stability score | + | ++ | ++++ |

The results show that sample 109 that contains the acid form of THC is more stable compare to sample 107 where pure THC has been used.

## Example 5

**Preparation of E-liquid containing THCA with a constant purity**

Table 4. Composition of E-liquid with 5% content of THCA.

| Raw Material | 110 | 111 | 112 | 113 |
|---|---|---|---|---|
| THCA (sample 104) | 5% | 5% | 5% | 5% |
| PG | 90% | 45% | 72% | 68% |
| VG | - | 45% | 18% | 17% |
| Flavour | 5% | 5% | 5% | 10% |

(continued)

| Raw Material | 110 | 111 | 112 | 113 |
|---|---|---|---|---|
| Total | 100 | 100 | 100 | 100 |

| The active ingredient THCA = 99% THCA; The raw material PG = Propylene glycol, VG = vegetables glycerin. Flavor may for example be pepper mint flavor. THCA could be replaced with CBDA or be in combination with CBDA. |
|---|

The E-liquid is prepared as described in example 2.

To achieve a stable homogenous mixture, it is important to have an optimal ratio between PG and VG. The preferred samples are sample no. 112 and sample no. 113 where a better taste profile has been achieved with the 10% flavor in sample no. 113. The ratio of PG/VG give the best mixture with 80/20w/w%.

**Example 6**

**Preparation of cannabinoid formulation with different cannabinoids and different purities**

Table 5. Composition of E-liquid with 5% content of THCA and in combination with CBDA. The active ingredient THCA vary from 90%-99% THCA; The raw material PG = Propylene glycol, VG = vegetables glycerin. Flavor may for example be pepper mint flavor. THCA could be replaced with CBDA.

| Raw Material | 114 | 115 | 116 | 117 |
|---|---|---|---|---|
| THCA (90%) | 5% | - | - | - |
| THCA (95%) | - | 5% | - | - |
| THCA (99%) | - | - | 5% | 5% |
| CBDA (99%) | - | - | - | 5% |
| PG | 68% | 68% | 68% | 64% |
| VG | 17% | 17% | 17% | 16% |
| Flavour | 10% | 10% | 10% | 10% |
| Total | 100 | 100 | 100 | 100 |

The samples 114-117 have been stored 1 month at 40°C/75% RH and the content of THCA has been analyzed by HPLC.

The stability results are listed in table 6. Stability score goes from + up to +++++, the sum of + means increased stability (1+ = low stability and 5+ = high stability).

Table 6. Stability results of E-liquid after 1 month storage at 40°C/75% RH.

| Stability results of E-liquid - 1 month at 40°C/75% RH | | | | |
|---|---|---|---|---|
| | 114 | 115 | 116 | 117 |
| Stability evaluation | ++++ | ++++ | ++++ | ++++ |

The results show that sample 114-117 have the same stability results. The stability is not affected by using different purity of the cannabinoids-acids.

**Example 7**

**Preparation of cannabinoid formulation with different concentrations when using THCA (99%):**

Table 7. Composition of E-liquid with different dosage of THCA. The active ingredient THCA = 99% THCA; The raw material PG = Propylene glycol, VG = vegetables glycerin. Flavor may for example be pepper mint flavor. THCA could be replaced with CBDA or in combination.

| Raw Material | 118 | 119 | 120 | 121 | 122 | 123 |
|---|---|---|---|---|---|---|
| THCA (99%) | 2% | 5% | 10% | 15% | 20% | 25% |
| PG | 70% | 68% | 64% | 60% | 56% | 52% |
| VG | 18% | 17% | 16% | 15% | 14% | 13% |
| Flavour | 10% | 10% | 10% | 10% | 10% | 10% |
| Total | 100 | 100 | 100 | 100 | 100 | 100 |

The samples 118-123 have been stored 1 month at 40°C/75% RH and the content of THCA has been analyzed by HPLC.

The stability results are listed in table 6. Stability score goes from + up to +++++, the sum of + means increased stability (1+ = low stability and 5+ = high stability).

Table 8. Stability results of E-liquid after 1 month storage at 40°C/75% RH.

| Stability results of E-liquid - 1 month at 40°C/75% RH | | | | | | |
|---|---|---|---|---|---|---|
| | 118 | 119 | 120 | 121 | 122 | 123 |
| Stability evaluation | ++++ | ++++ | ++++ | ++++ | ++++ | ++++ |

The results show that sample 118-123 have the same stability results. The stability is not affected by using different dosage of THCA purity of the cannabinoids-acids.

**Example 8**

**Converting the THCA in the cartomizer into the active THC**

Sample 119 containing 5% THCA and sample 121 containing 15% THCA, has been tested in a standard device to test the efficiency of the conversion. The device has a cartomizer of 1 ml and a coil temperature of approx. 220°C when using at battery of 180mAh.

Table 9. Conversion of THCA into the active THC.

| Conversion of THC-acid into THC | | |
|---|---|---|
| | 119 | 121 |
| Conversion of THCA | Yes | Yes |

The results show that both samples convert the acid form into the active THC form during vaping.

**Claims**

1. A liquid cannabinoid composition (LCC),
   said liquid cannabinoid composition (LCC) comprising
   a vaporizer carrier liquid (VCL), and
   a tetrahydrocannabinolic compound and/or a cannabidiolic compound,
   wherein at least 95 percent by weight of said tetrahydrocannabinolic compound and/or cannabidiolic compound is present in acid form.

2. The liquid cannabinoid composition (LCC) according to claim 1, wherein at least 98 percent by weight of said tetrahydrocannabinolic compound and/or cannabidiolic compound is present in acid form.

3. The liquid cannabinoid composition (LCC) according to any of claims 1-2, wherein said tetrahydrocannabinolic compound and/or a cannabidiolic compound is present in an amount of between 0.01 and 25 percent by weight of said liquid cannabinoid composition, such as between 1 and 10 percent by weight of the liquid cannabinoid composition.

4. The liquid cannabinoid composition (LCC) according to any of claims 1-3, wherein said liquid cannabinoid composition (LCC) comprises non-cannabinoid terpenoids in an amount of less than 5 percent by weight.

5. The liquid cannabinoid composition (LCC) according to any of claims 1-4, wherein said liquid cannabinoid composition (LCC) comprises non-cannabinoid terpenoids, flavonoids, and phytosterols in a total amount of less than 5 percent by weight.

6. The liquid cannabinoid composition (LCC) according to any of claims 1-5, wherein said vaporizer carrier liquid (VCL) is chosen from the group consisting of water; alcohols, such as ethanol, propylene glycol, polyethylene glycol such as PEG 400, glycerol, and other similar alcohols; and mixtures or combinations thereof.

7. The liquid cannabinoid composition (LCC) according to any of claims 1-6, wherein the liquid cannabinoid composition (LCC) has a boiling point of between 80 and 400 degrees Celsius, such as between 100 and 250 degrees Celsius.

8. The liquid cannabinoid composition (LCC) according to any of claims 1-7, wherein the liquid cannabinoid composition (LCC) is for pulmonary and/or oromucosal administration.

9. The liquid cannabinoid composition (LCC) according to any of claims 1-8, wherein said liquid cannabinoid composition (LCC) comprises 0.01 - 10 percent by weight of flavoring, such as 0.01 - 5 percent by weight of flavoring, 0.01 - 0.5 percent by weight of flavoring.

10. The liquid cannabinoid composition (LCC) according to any of claims 1-9 for use as a medicament.

11. The liquid cannabinoid composition (LCC) according to any of claims 1-9 for use in alleviation of pain, such as neurotic pain or cancer-related pain.

12. The liquid cannabinoid composition (LCC) according to any of claims 1-9 for therapeutic use in mitigation of appetite deficiency.

13. A cannabinoid container (CC) comprising the liquid cannabinoid composition (LCC) according to any of claims 1-9.

14. A personal vaporizer (PV) comprising a cannabinoid container (CC) according to claim 13.

15. The personal vaporizer (PV) according to claim 14, wherein the personal vaporizer (PV) further comprises a heating element (HE) for heating the liquid cannabinoid composition (LCC) to obtain aerosols, wherein at least 50 percent by weight of said tetrahydrocannabinolic compound and/or cannabidiolic compound is present in active form in said aerosols.

**Patentansprüche**

1. Flüssige Cannabinoid-Zusammensetzung (LCC),
wobei die flüssige Cannabinoid-Zusammensetzung (LCC) umfasst
eine Verdampfer-Trägerflüssigkeit (VCL), und
eine Tetrahydrocannabinol-Verbindung und/oder eine Cannabidiol-Verbindung,
wobei mindestens 95 Gewichtsprozent der Tetrahydrocannabinol-Verbindung und/oder der Cannabidiol-Verbindung in Säureform vorliegen.

2. Flüssige Cannabinoid-Zusammensetzung (LCC) nach Anspruch 1, wobei mindestens 98 Gewichtsprozent der Tetrahydrocannabinol-Verbindung und/oder der Cannabidiol-Verbindung in Säureform vorliegen.

3. Flüssige Cannabinoid-Zusammensetzung (LCC) nach einem der Ansprüche 1-2, wobei die Tetrahydrocannabinol-Verbindung und/oder eine Cannabidiol-Verbindung in einer Menge von zwischen 0,01 und 25 Gewichtsprozent der flüssigen Cannabinoid-Zusammensetzung, wie etwa zwischen 1 und 10 Gewichtsprozent der flüssigen Cannabinoid-Zusammensetzung vorliegen.

4. Flüssige Cannabinoid-Zusammensetzung (LCC) nach einem der Ansprüche 1-3, wobei die flüssige Cannabinoid-Zusammensetzung (LCC) Nicht-Cannabinoid-Terpenoide in einer Menge von weniger als 5 Gewichtsprozent umfasst.

5. Flüssige Cannabinoid-Zusammensetzung (LCC) nach einem der Ansprüche 1-4, wobei die flüssige Cannabinoid-Zusammensetzung (LCC) Nicht-Cannabinoid-Terpenoide, Flavonoide, und Phytosterole in einer Gesamtmenge von weniger als 5 Gewichtsprozent umfasst.

6. Flüssige Cannabinoid-Zusammensetzung (LCC) nach einem der Ansprüche 1-5, wobei die Verdampfer-Trägerflüssigkeit (VCL) ausgewählt ist aus der Gruppe bestehend aus Wasser; Alkoholen, wie etwa Ethanol, Propylenglycol, Polyethylenglycol, wie etwa PEG 400, Glycerol, und anderen ähnlichen Alkoholen; und Mischungen oder Kombinationen davon.

7. Flüssige Cannabinoid-Zusammensetzung (LCC) nach einem der Ansprüche 1-6, wobei die flüssige Cannabinoid-Zusammensetzung (LCC) einen Siedepunkt von zwischen 80 und 400 Grad Celsius, wie etwa zwischen 100 und 250 Grad Celsius aufweist.

8. Flüssige Cannabinoid-Zusammensetzung (LCC) nach einem der Ansprüche 1-7, wobei die flüssige Cannabinoid-Zusammensetzung (LCC) zur Anwendung in der Lunge und/oder der Mundhöhle bestimmt ist.

9. Flüssige Cannabinoid-Zusammensetzung (LCC) nach einem der Ansprüche 1-8, wobei die flüssige Cannabinoid-Zusammensetzung (LCC) 0,01 - 10 Gewichtsprozent Aromastoff, wie etwa 0,01 - 5 Gewichtsprozent Aromastoff, 0,01 - 0,5 Gewichtsprozent Aromastoff umfasst.

10. Flüssige Cannabinoid-Zusammensetzung (LCC) nach einem der Ansprüche 1-9 zur Verwendung als ein Arzneimittel.

11. Flüssige Cannabinoid-Zusammensetzung (LCC) nach einem der Ansprüche 1-9 zur Verwendung in der Linderung von Schmerzen, wie etwa Nervenschmerzen oder krebsbedingten Schmerzen.

12. Flüssige Cannabinoid-Zusammensetzung (LCC) nach einem der Ansprüche 1-9 zur therapeutischen Verwendung in der Bekämpfung von Appetitmangel.

13. Cannabinoid-Behälter (CC), der die flüssige Cannabinoid-Zusammensetzung (LCC) nach einem der Ansprüche 1-9 umfasst.

14. Persönlicher Verdampfer (PV), der einen Cannabinoid-Behälter (CC) nach Anspruch 13 umfasst.

15. Persönlicher Verdampfer (PV) nach Anspruch 14, wobei der persönliche Verdampfer (PV) weiter ein Heizelement (HE) zum Erhitzen der flüssigen Cannabinoid-Zusammensetzung (LCC) umfasst, um Aerosole zu erhalten, wobei mindestens 50 Gewichtsprozent der Tetrahydrocannabinol-Verbindung und/oder der Cannabidiol-Verbindung in den Aerosolen in aktiver Form vorliegen.

**Revendications**

1. Composition de cannabinoïde liquide (LCC),
   ladite composition de cannabinoïde liquide (LCC) comprenant
   un liquide vecteur de vaporisateur (VCL), et
   un composé tétrahydrocannabinolique et/ou un composé cannabidiolique,
   dans laquelle au moins 95 pour cent en poids dudit composé tétrahydrocannabinolique et/ou dudit composé cannabidiolique est présent sous forme acide.

2. Composition de cannabinoïde liquide (LCC) selon la revendication 1, dans laquelle au moins 98 pour cent en poids dudit composé tétrahydrocannabinolique et/ou dudit composé cannabidiolique est présent sous forme acide.

**3.** Composition de cannabinoïde liquide (LCC) selon l'une quelconque des revendications 1-2, dans laquelle ledit composé tétrahydrocannabinolique et/ou un composé cannabidiolique est présent en une quantité entre 0,01 et 25 pour cent en poids de ladite composition de cannabinoïde liquide, par exemple entre 1 et 10 pour cent en poids de la composition de cannabinoïde liquide.

**4.** Composition de cannabinoïde liquide (LCC) selon l'une quelconque des revendications 1-3, dans laquelle ladite composition de cannabinoïde liquide (LCC) comprend des terpénoïdes non cannabinoïdes en une quantité inférieure à 5 pour cent en poids.

**5.** Composition de cannabinoïde liquide (LCC) selon l'une quelconque des revendications 1-4, dans laquelle ladite composition de cannabinoïde liquide (LCC) comprend des terpénoïdes non cannabinoïdes, des flavonoïdes et des phytostérols en une quantité totale inférieure à 5 pour cent en poids.

**6.** Composition de cannabinoïde liquide (LCC) selon l'une quelconque des revendications 1-5, dans laquelle ledit liquide vecteur de vaporisateur (VCL) est choisi dans le groupe constitué d'eau ; d'alcools, par exemple d'éthanol, de propylène glycol, de polyéthylène glycol, par exemple de PEG 400, de glycérol, et d'autres alcools similaires ; et de mélanges ou de combinaisons de ceux-ci.

**7.** Composition de cannabinoïde liquide (LCC) selon l'une quelconque des revendications 1-6, dans laquelle la composition de cannabinoïde liquide (LCC) présente un point d'ébullition entre 80 et 400 degrés Celsius, par exemple entre 100 et 250 degrés Celsius.

**8.** Composition de cannabinoïde liquide (LCC) selon l'une quelconque des revendications 1-7, dans laquelle la composition de cannabinoïde liquide (LCC) est pour une administration pulmonaire et/ou oromucosale.

**9.** Composition de cannabinoïde liquide (LCC) selon l'une quelconque des revendications 1-8, dans laquelle ladite composition de cannabinoïde liquide (LCC) comprend 0,01-10 pour cent en poids d'aromatisant, par exemple 0,01-5 pour cent en poids d'aromatisant, 0,01-0,5 pour cent en poids d'aromatisant.

**10.** Composition de cannabinoïde liquide (LCC) selon l'une quelconque des revendications 1-9 pour une utilisation en tant que médicament.

**11.** Composition de cannabinoïde liquide (LCC) selon l'une quelconque des revendications 1-9 pour une utilisation dans le soulagement d'une douleur, par exemple une douleur névrotique ou une douleur liée à un cancer.

**12.** Composition de cannabinoïde liquide (LCC) selon l'une quelconque des revendications 1-9 pour une utilisation thérapeutique dans l'atténuation d'un manque d'appétit.

**13.** Récipient de cannabinoïde (CC) comprenant la composition de cannabinoïde liquide (LCC) selon l'une quelconque des revendications 1-9.

**14.** Vaporisateur personnel (PV) comprenant un récipient de cannabinoïde (CC) selon la revendication 13.

**15.** Vaporisateur personnel (PV) selon la revendication 14, dans lequel le vaporisateur personnel (PV) comprend en outre un élément chauffant (HE) pour chauffer la composition de cannabinoïde liquide (LCC) pour obtenir des aérosols, dans lequel au moins 50 pour cent en poids dudit composé tétrahydrocannabinolique et/ou dudit composé cannabidiolique est présent sous forme active dans lesdits aérosols.

EP 3 558 278 B1

Fig. 1

Fig. 2

17

EP 3 558 278 B1

*Fig. 3*

18